# EUROPEAN PATENT SPECIFICATION

(11) **EP 1 728 078 B1**
(45) Date of publication and mention of the grant of the patent: **25.08.2010**
(21) Application number: 05725781.8
(22) Date of filing: 16.03.2005
(51) Int. Cl.: G01N 33/543, C12Q 1/68

(54) **PROCESS AND REAGENTS FOR EXTRACTION OF RNA FROM FRACTIONATED BLOOD LEUKOCYTES**
PROZESS UND REAGENZIEN FÜR EXTRAKTION VON RNS VON FRAKTIONIERTEN BLUTLEUKOZYTEN
PROCEDE ET REACTIFS POUR L'EXTRACTION D'ARN A PARTIR DE LEUCOCYTES DU SANG FRACTIONNES

(30) Priority: 16.03.2004 US 801982
(43) Date of publication of application: 06.12.2006
(73) Proprietor: Ambion, Inc., Austin, TX 78744-1832 (US)
(72) Inventor: GOLDRICK, Marianna, Austin, TX 78704 (US)
(74) Representative: Muir, Benjamin M. J.
(86) International application number: PCT/US2005/008826
(87) International publication number: WO 2005/090984

(56) References cited:
- WO-A-01/11362
- WO-A-94/17209
- US-A- 4 925 572
- HÄMÄLÄINEN M M ET AL: "Major interference from leukocytes in reverse transcription-PCR identified as neurotoxin ribonuclease from eosinophils: detection of residual chronic myelogenous leukemia from cell lysates by use of an eosinophil-depleted cell preparation." CLINICAL CHEMISTRY. APR 1999, vol. 45, no. 4, April 1999 (1999-04), pages 465-471, XP002329130 ISSN: 0009-9147

## Description

### 1. Field of the Invention

This invention relates to processes for fractionating white blood cells (also called "WBCs" or "leukocytes") from blood, for enhancing the stability of RNA molecules in the fractionated cells, and for extracting RNA from the fractionated cells. The invention further relates to the analysis of mRNA, including mRNA expression patterns, using techniques such as expression profiling and RT-PCR. The invention further relates to analysis of small RNAs including micro-RNAs ("miRNAs"), siRNAs, etc.

### 2. Description of Related Art

Blood is the most accessible human tissue from which appreciable amounts of RNA can be recovered. It is also the most expendable, since a substantial amount can be removed with no ill effects and it is rapidly regenerated. Blood also has a unique ability to report events that are widely disseminated, since the design of the circulatory system ensures that blood cells are in intimate contact with tissues in essentially all parts of the body. This means that circulating leukocytes have the potential to act as sentinel cells for the surveillance of distant tissues affected by infection, cancer, inflammation, and genetic and metabolic diseases (Whitney *et al.,* 2002; Alcorta *et al.,* 2002). For example a recent report describes changes in mRNA patterns in density gradient-fractionated lymphocytes that correlate with central nervous system pathologies including ischemic stroke and seizure in a rat model system (Tang *et al.,* 2001).

The promise of high-density microarrays to identify global changes in mRNA expression that correlate with diagnosis, prognosis, and treatment outcome is now being realized. Expression profiling assays have identified differences in mRNA patterns that are associated with inflammatory and metastatic disease, exposure to toxic and infectious agents, and ionizing radiation. Once microarray analysis has identified the key mRNA changes, simpler, cheaper, and more sensitive assays, such as quantitative RT-PCR, may be developed for clinical purposes. To facilitate both the discovery phase and the clinical implementation of mRNA expression profiling, methods are needed for rapidly stabilizing the RNA in clinical samples, especially in human blood.

However, as discussed below, there are several technical challenges to using blood as source tissue for mRNA expression profiling.

### Challenges in the Isolation of RNA from Blood

One initial challenge is that the concentration of actively metabolizing cells (*i*.*e*., cells that contain mRNA) in blood is low. The concentration of mRNA in blood is therefore relatively low. Less than 50% of blood by weight and volume is comprised of cells (>50% of blood is non-cellular plasma), and of the cellular fraction, only ∼ 0.1-0.2% is comprised of leukocytes, the remainder being mostly mature red blood cells ("RBCs" or "erythrocytes"), which do not generally express mRNA. The vast majority (>99%) are mature red cells, which contain no appreciable mRNA but have high concentrations of heme. Heme is a potent inhibitor of enzymatic reactions including reverse transcription and PCR. Since virtually all protocols for extraction of cellular RNA require disruption of the tissue in several volumes of extraction reagent (typically ∼ 3 - 10 volumes), the use of unfractionated whole blood as source tissue results in a further dilution of the sample and complicates RNA recovery. For example, processing of 10 ml of whole blood using conventional procedures (Choczymski/Sacchi, Trizol) would require the addition of 100 ml (10 volumes) of lysis solution. The resulting sample volume is impractical for use in organic extraction and solid-phase extraction protocols.

Another challenge is that blood contains high concentrations of intracellular and extracellular ribonucleases (Moenner *et al.,* 1997) making recovery of intact mRNA difficult.

An important complication of expression profiling in whole blood is the presence of high numbers (relative to number of leukocytes) of immature RBC ("reticulocytes"), which contain high levels of mRNA for α and β globin. When RNA is extracted from whole blood and amplified for use in microarray expression profiling assays, the product of globin mRNA predominates in the amplified material and results in dramatically reduced sensitivity (Affymetrix Technical Notes, "Blood RNA Isolation Methods for GeneChip Expression Analysis"; "An Analysis of Blood Processing Methods to Prepare Samples for GeneChip Expression Profiling"). This globin mRNA contamination compromises the use of the RNA as input for microarray profiling.

### Methods for Fractionating WBCs from Whole Blood

For practical reasons, as set forth above, whole blood is often fractionated to concentrate the nucleated cells (*i*.*e*., the WBCs) prior to RNA extraction or immunoselection. Removing plasma and red cells eliminates many of the nucleases and inhibitors from blood and reduces the sample volume by at least ten-fold, which allows RNA extraction to be carried out in microfuge tubes (<2 ml) instead of larger vessels. Also, most protocols for immunoselection of leukocyte subsets are more efficient and cost-effective when carried out on fractionated leukocytes rather than whole blood.

Density gradient centrifugation is probably the most common method for fractionating whole blood; a blood sample (several milliliters) is diluted and then layered onto a viscous polysaccaride polymer (sold commercially as Ficoll-Hypaque) and centrifuged for ∼ 30 minutes under conditions that cause the red blood cells and granulocytes to sediment below the polymer, while the lymphocytes (B-cells and T-cells) and other mononuclear cells (including monocytes and leukemic blasts) form a band within the polymer (Theophilus, 1998). The band is withdrawn by aspiration and the sample is diluted to allow the lymphocytes to be re-pelleted, then the cells are washed in PBS, collected again by centrifugation, and then either used directly for RNA isolation or expanded by tissue culture prior to RNA extraction. Although high-quality RNA can generally be recovered from blood cells processed in this way, the procedure is time-consuming and labor-intensive, and the extensive manipulation required may result in significant changes in mRNA profiles prior to RNA extraction and analysis. There has been only one study to date that described changes in mRNA expression between whole blood and lymphocytes fractionated via density gradient (Hartel *et al.,* 2001). That group found increases in several cytokine mRNAs (IL-4, IL-2, TNF-alpha) in Ficoll-fractionated lymphocytes.

A variation on Ficoll separation is a commercially available device ("CPT tubes", BD) that allows the blood to be drawn directly into the tube containing the polymer gel. After centrifugation, the plasma and lymphocytes are decanted, residual cells are washed from the sides of the tube, the cells are diluted and re-pelleted, and then usually put into tissue culture prior to RNA isolation. No reagent is included in the CPT tube to stabilize the expression profile during these manipulations.

Another method for fractionating leukocytes for subsequent RNA extraction is to selectively lyse the RBCs in several volumes of ammonium chloride solution or other RBC lysis reagent and recover the leukocytes by centrifugation (Duvigneau *et al.,* 2003). This procedure requires inconvenient increases in sample volume and subjects the sample to highly unphysiological conditions that may alter the expression profile, and results in a low yield and quality of RNA recovered. Another method for fractionating leukocytes is to centrifuge the anticoagulated whole blood at low speed (∼ 2,000 x g) for ∼ 15 minutes to separate the "buffy coat" (*i*.*e*., the WBCs) at the interface between the plasma and the packed red cells. The buffy coat can be collected by aspiration after removing the plasma. An advantage of this method is that the leukocytes are fractionated under conditions that are arguably closer to physiological conditions than the methods described above. However, collection of buffy coat is still a time-consuming process that requires centrifugation and other manipulations that may alter mRNA expression patterns. Also, it is messy and labor-intensive and carries a risk of exposing healthcare workers to blood-borne pathogens. Further, the buffy coat fraction containing leukocytes is highly contaminated with RBCs and reticulocytes, and thus also contaminated with globin mRNA.

Pall Corporation manufactures leukocyte depletion matrices that are used in blood transfusion therapy to deplete donor immune cells and thus reduce problems of graft-vs-host disease in recipients. Two reports in the literature describe retrieval of leukocyte depletion filter-captured leukocytes from blood bank by-products (Weitkamp and Crowe, 2001; Ebner *et al*., 2001). Subsets of cells (dendritic cells or B-cells) were then selected using density gradient fractionation and antibody-coated magnetic beads, and the cells recovered were used to establish viable cell lines. However, such filters have evidently not been used in the context of recovery of RNA from primary WBCs.

### Methods for Extracting RNA from Whole Blood

Most methods for extracting RNA from whole blood or fractionated leukocytes involve an initial lysis of the sample in guanidinium thiocyanate. For example, one such method involves solid-phase extraction onto a silica matrix wherein a blood sample is lysed in a guanidinium solution, and the cell lysate is mixed with ethanol and applied to the silica filter, which binds RNA. Other methods are based on lysis of whole blood in cationic detergents (Macfarlane and Dahle, 1997) or in lithium chloride/urea solutions. Commercial products for isolating RNA from blood include the Ambion RiboPure-Blood kit (Ambion) and the PAXgene system (PreAnalytix). A limitation of all of the above methods for isolating RNA from blood is that all the cells, including RBCs and reticulocytes, are lysed in the first step. This can result in contamination of the leukocyte RNA with globin mRNAs and precludes subsequent fractionation of leukocyte subsets. A method of reducing such contamination would be beneficial.

### RNA Requirements for Expression Profiling

The requirements for RNA for use in microarray analysis and RT-PCR are somewhat different than those for historically used RNA analysis methods such as Northern blots and nuclease protection assays. The most significant difference is that rather than relying only on the ability of the sample RNA to hybridize to a probe, the current methods require that the RNA serve as an efficient substrate for reverse transcription. Reverse transcription converts the sample RNA into cDNA in order to incorporate fluorescent labels or other detectable moieties (for microarray analysis), or to generate a cDNA template for subsequent amplification by thermostable DNA polymerase (for RT-PCR). An important requirement for the sample RNA is that it must not contain contaminants that would inhibit enzymatic reactions. In the case of RNA extraction from blood, it is important that the RNA be free from contamination with heme, a well-known inhibitor of reverse transcription and DNA polymerase. In terms of amounts of RNA required for expression studies, microarray analysis has historically required relatively large amounts, ranging from ∼ 2 - 20 µg. However, an emerging trend is to use much smaller amounts of total RNA. This is made possible by using linear amplification strategies to enzymatically increase the amount of input RNA, and/or by using more sensitive detection methods that result in signal amplification. These technical advances have made it possible to carry out microarray experiments using only a few cells (Xiang *et al.,* 2003). These considerations suggest that microarray analysis on uncultured primary leukocyte subclasses will be feasible.

### Expression Profiling of Blood

It has been proposed that determining characteristic patterns of gene expression in circulating peripheral blood cells may prove broadly useful for noninvasive diagnostics (Staudt and Brown, 2000). Most microarray experiments that have been carried out using blood as the sample have used density-gradient-fractionated peripheral blood mononuclear cells (PBMCs), which are often expanded in tissue culture prior to RNA extraction. Fractionation of PBMCs *via* density gradient centrifugation is, however, a labor-intensive and time-consuming process that carries a high risk of exposing workers to blood-borne pathogens (since it is carried out in open vessels) and permits only several milliliters of blood to be used per preparation. An even more serious drawback to density gradient fractionation and culturing the recovered cells is that the manipulations required may result in changes in the endogenous mRNA levels, which will compromise interpretation of mRNA expression profiling assays. Another drawback is that density gradient fractionation fails to recover a major subset of mature WBCs (those of the myeloid lineage) from whole blood.

In order to discover mRNA patterns in circulating WBCs that correlate with diagnosis, prognosis, and treatment outcome, it is important to freeze the mRNA pattern in the cells prior to RNA extraction, so that it accurately reflects the relative levels of endogenous gene expression. Numerous reports document changes in mRNA levels in unstabilized blood samples, as described below.

Any changes in mRNA levels in blood cells that occur during sample collection and handling will interfere with the quantitative detection of specific mRNA changes for diagnostic purposes. There are many reports in the literature of changes in mRNAs in untreated *ex vivo* blood. For example, adherence of monocytes to the plastic walls of tubes used for blood collection causes induction of mRNAs for proinflammatory cytokines (Haskill *et al.,* 1988). Significant increases were reported in mRNA expression of several cytokines including IL-2, IL-4, and tumor necrosis factor alpha, that were associated with Ficoll density gradient separation and with other blood collection parameters (Hartel *et al.,* 2001). Lag times of greater than one hour between sample collection and processing were reported to cause alterations in some but not all cytokines tested in a porcine blood system (Duvigneau *et al.,* 2003). Increases in levels of several cytokine mRNAs in human whole blood after *ex vivo* storage were also reported (Pahl and Brune, 2002). Based on real-time RT-PCR assays, Stordeur *et al.* (2002) demonstrated that interferon γ mRNA is rapidly degraded in *ex vivo* blood with a half-life of about one hour. Probably the most extensive study dealing with mRNA changes in untreated whole blood during *ex vivo* storage is a recent report by Rainen *et al*. (2002). This group compared levels of 25 mRNAs (including cytokines, transcription factors, apoptosis-related genes, tumor suppressor genes, *etc*.) in RNA isolated from whole blood from a single donor stored for increasing times at room temperature in either standard EDTA-anticoagulated vacutainer tubes or in the cationic detergent solution used in the PAXgene blood RNA isolation system (described above ). The mRNA levels were determined by qRT-PCR and expressed relative to that of 18S ribosomal RNA. The mRNA levels from RNA isolated using the two methods at each *ex vivo* time point (4 hours, 8 hours, 24 hours, 3 days, and 5 days) were then compared to the levels seen in RNA isolated from EDTA blood samples immediately after blood collection. The extent of mRNA changes was greater for the EDTA blood compared to the blood stabilized in the PAXgene system, although changes were observed for both conditions. Interestingly, the relative levels of some mRNAs (*e*.*g*., interleukin 8, c-jun oncogene) were observed to increase over time in the EDTA tubes, while others (*e*.*g*., caspase 1, heat shock protein 70) were seen to decrease. Overall, increases in relative mRNA levels were as likely to occur as decreases over time in EDTA anticoagulated blood, and increases were more often observed than decreases for the PAXgene system.

All of the above reports exemplify that gene-specific changes in mRNA levels occur in unstabilized *ex vivo* blood over relatively short time scales. Such changes will compromise the discovery of mRNA expression patterns associated with disease and prognosis.

For the above reasons, and others, there is a need for methods for immediately fractionating the WBC population from whole blood, and for stabilizing the mRNA expression profile in the fractionated leukocytes, in order to facilitate extraction of mRNA from blood for use in clinical assays.

### SUMMARY OF THE INVENTION

The current invention relates generally to in-vitro methods for rapid fractionation of WBCs from whole blood using a proprietary leukocyte depletion filter, and for subsequent stabilization of the RNA patterns in the fractionated cells. The invention further relates to methods of extracting the RNA, and in some cases DNA, from the fractionated cells and using such nucleic acids in any of a variety of molecular biology procedures including, for example, expression profiling and RT-PCR.

According to a first aspect, the invention provides in-vitro methods of obtaining a leukocyte lysate comprising RNA, which methods comprise fractionating leukocytes from whole blood by passing the blood through a leukocyte depletion filter (also referred to herein as a leukocyte depletion matrix) and lysing the fractionated leukocytes to obtain a lysate comprising RNA. In some embodiments the leukocytes are comprised on the matrix at the time they are lysed, i.e., there is no steps of eluting or removing the leukocytes from the matrix prior to lysing are needed. However, there are certainly embodiments of the invention in which it is possible to flush the leukocytes from the depletion matrix prior to lysis.

In some embodiments, the leukocyte comprising matrix is stored for a period of time prior to lysis of the leukocytes. For example, this storage may be minutes, hours, days, weeks, or years. In some embodiments of the invention, treatment of the leukocyte comprising matrix with an RNA preservation solution prior to storage is employed.

Lysis of the fractionated leukocytes is often accomplished with a leukocyte lysis solution, although other methods of lysis known to those of skill may be employed. Such lysis solutions often contain a detergent, for example, Triton X-100, Tween-20, SDS (sodium dodecyl sulfate), sarcosyl, deoxycholic acid, etc. The lysis solution may also contain a chaotropic agent, for example, a guanidinium salt such as guanidinum thiocyanate. The lysis solution may additionally comprise a protease for example proteinase K may be included. For the purposes of this specification the terms "protease" and "proteinase" are used interchangeably. The lysis solution may also comprise a ribonuclease inhibitor, such as those described in: (i) U.S. provisional application entitled "Nuclease Inhibitors for Use In Biological Applications" by Latham *et al.,* filed on February 25, 2004; and (ii) U.S. non-provisional application entitled "Improved Nuclease Inhibitor Cocktail" by Latham *et al.,* filed on February 25, 2004, which is a continuation-in-part application of co-pending U.S. Application No. 10,675,860 filed September 30, 2003, which is a continuation of Application No. 09/669,301 filed September 25, 2000, now U.S. Patent 6,664,379, which claims the benefit of U.S. Provisional Application No. 60/155,874, filed September 24, 1999.

Some preferred embodiments comprise extracting the RNA from the lysate. In many cases, this extraction is performed via an organic extraction. In some preferred embodiments, the organic extraction is a phenol/chloroform extraction. These methods may also comprise isolating DNA from the lysate. Of course, in the context of isolating DNA from the lysate, either in combination with RNA or alone, those of skill will typically not wish to employ the procedures taught herein for reducing DNA contamination in those protocols where only RNA isolation is desired. Preferred embodiments comprise, prior to lysis, treating the fractionated leukocytes with an RNA preservation composition comprising a salt that infiltrates the leukocytes and increases the stability of the RNA compared to the RNA in cells not treated with the preservation composition. Such an RNA preservation medium is the Ambion product RNA*later*®, which is covered by U.S. Patents No. 6,204,375 and 6,528,641. In some preferred methods, the salt is a sulfate salt, for example but not limited to ammonium sulfate. In some embodiments, the final salt concentration in the preservation composition is between 10 g/100 ml and a saturating concentration; between 20 g/100 ml and the saturating concentration of the salt; and/or between 30 g/100 ml and 80 g/100 ml. The RNA preservation composition may comprises at least two, three, four, or more salts. In some preferred embodiments, the fractionated leukocytes are comprised on the leukocyte depletion matrix and the matrix is contacted with the RNA preservation composition. These embodiments may then involve extracting RNA from the fractionated leukocytes with an organic extraction. An advantage in this regard is that the extracted RNA has less DNA contamination than would RNA extracted from fractionated leukocytes that were not treated with the RNA preservation medium. For example, it is possible to obtain 95%, 90%, 85%, 80%, 75%, 70%, 60%, 50%, 40%, 30%, and/or 25% or more less DNA contamination in this manner, including but not limited to a range of percentage reduction between any of these specific points.

In some specific embodiments, the invention involves: fractionating leukocytes from blood by capturing them with a leukocyte depletion matrix; lysing the fractionated leukocytes to produce a lysate; extracting the lysate with an organic solution to form organic and aqueous phases; separating the organic and aqueous phases after the organic phase has been absorbed into the matrix; and isolating RNA from the aqueous phase.

In other more specific embodiments, some methods of the invention comprise: fractionating leukocytes from blood by capturing them with a leukocyte depletion matrix; treating the fractionated leukocytes with an RNA preservation composition comprising a salt that infiltrates the leukocytes, increasing the stability of the RNA; lysing the fractionated leukocytes to produce a lysate; extracting the lysate with an organic solution to form organic and aqueous phases; separating the organic and aqueous phases; and isolating RNA from the aqueous phase.

Other embodiments comprise: fractionating leukocytes from blood by capturing them with a leukocyte depletion matrix; treating the fractionated leukocytes with an RNA preservation composition comprising a salt that infiltrates the leukocytes, increasing the stability of the RNA; lysing the fractionated leukocytes to produce a lysate; and isolating RNA from the lysate.

In a further embodiment of the invention, fractionated leukocytes can be treated in such a way as to reduce the levels of contaminating erythrocytes and reticulocytes prior to lysis of the leukocytes. Reduced contamination of RBCs can be of particular benefit in some uses of the invention since the reticulocytes contain high concentrations of mRNA coding for α globin and β globin. Thus, removal of reticulocytes from leukocyte preparations reduces the amount of contaminating α and β globin mRNA in the resulting leukocyte RNA preparations. It is contemplated that fractionated leukocytes may be washed with a solution to elute residual reticulocytes and erythrocytes. Reagents that may be useful as to elute residual reticulocytes and erythrocytes, referred to herein as "RBC elution buffers", include but are not limited to physiological saline solutions such as phosphate-buffered saline (PBS), and low ionic strength solutions such as distilled water. It is also contemplated that reticulocyte contamination may be reduced by treating fractionated leukocytes with a RBC lysis solution known by those of skill in the art to effectively lyse reticulocytes as well as erythrocytes. For example such RBC lysis solutions can be but are not limited to, solutions comprising water, an ammonium chloride solution or an ammonium chloride/sodium bicarbonate solution.

In preferred embodiments, the invention involves: fractionating leukocytes from blood by capturing them with a leukocyte depletion matrix; treating the fractionated leukocytes with a RBC lysis solution; lysing the fractionated leukocytes to produce a lysate; extracting the lysate with an organic solution to form organic and aqueous phases; separating the organic and aqueous phases after the organic phase has been absorbed into the matrix; and isolating RNA from the aqueous phase.

In even more preferred embodiments, the invention involves: fractionating leukocytes from blood by capturing them with a leukocyte depletion matrix; treating the fractionated leukocytes with a RBC lysis solution; treating the fractionated leukocytes with an RNA preservation composition comprising a salt that infiltrates the leukocytes, increasing the stability of the RNA; lysing the fractionated leukocytes to produce a lysate; extracting the lysate with an organic solution to form organic and aqueous phases; separating the organic and aqueous phases after the organic phase has been absorbed into the matrix; and isolating RNA from the aqueous phase.

Further embodiments are defined as comprising: fractionating leukocytes from blood by capturing them with a leukocyte depletion matrix; lysing the fractionated leukocytes to produce a lysate; and isolating RNA from the lysate.

Some embodiments of the invention comprise assaying for the presence or quantity of one or more RNAs in the lysate. Such assaying may comprise any form of molecular biological assay suitable for assaying RNA, whether known at the time of the filing of this specification or developed later. Those of skill, in view of this specification will understand how to adapt the invention to such assays. Such assays may be used in the context of research and/or diagnostic protocols. Such assaying may comprise a Northern blot, RNAse protection assay, hybridization reaction, microarray analysis, or reverse transcriptase-polymerase chain reaction analysis. Embodiments comprising the use of reverse transcriptase-polymerase chain reaction, may be further defined as employing real-time RT-PCR or endpoint RT-PCR.

RNA obtained from lysates may also be assayed in protocols that comprise a microarray analysis. In some preferred embodiments, the microarray analysis may comprise the use of a cDNA array, spotted oligonucleotide array, or in-situ synthesized oligonucleotide array. As discussed herein, a benefit of some embodiments of the invention is that it allows for a reduction in the amount of α globin and β globin gene mRNA contamination from reticulocytes, greatly facilitating WBC RNA analysis, such as microarray analysis.

According to a second aspect, the invention provides a kit for extracting total RNA from leukocytes comprising: a leukocyte depletion filter (leukocyte depletion matrix); and a cell lysis solution for leukocytes as described above. In some embodiments, the leukocyte depletion matrix is comprised in a carrier adapted to allow blood to be passed through the matrix during use. Such a carrier can be adapted to be fitted to a syringe. The kits of some embodiments are adapted to function in a manner that allows whole blood to be moved from a closed container through the matrix and then, as leukocyte-depleted blood, into a further closed container. One or more containers may be included in the kit. The kits may also include other components including but not limited to one, two, or three of: an RBC lysis solution, an RNA preservation composition comprising a salt that infiltrates leukocytes and increases the stability of RNA in the leukocytes; an organic extraction reagent; and/or a solid-phase extraction matrix and reagents for washing the matrix to remove impurities before elution of the RNA.

The use of the word "a" or "an" when used in conjunction with the term "comprising" in the claims and/or the specification may mean "one," but it is also consistent with the meaning of "one or more," "at least one," and "one or more than one."

It is contemplated that any embodiment discussed in this specification can be implemented with respect to any method, kit, reagent, or composition of the invention, and *vice versa*. Furthermore, compositions of the invention can be used to achieve methods of the invention.

Throughout this application, the term "about" is used to indicate that a value includes the inherent variation of error for the device, the method being employed to determine the value, or the variation that exists among the study subjects.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1** Efficiency on capture of leukocytes on Pall LK4 leukocyte depletion matrix.
**FIG. 2** High-quality RNA recovered from LK4-fractionated leukocytes.
**FIG. 3A and FIG. 3B** High-quality RNA is shown by analysis on Agilent 2100 Bioanalyzer.
**FIG. 4** RNA is more stable in LK4-fractionated leukocytes which are subsequently treated with RNA*later* compared to RNA in untreated LK4-fractionated leukocytes..
**FIG. 5** RNA*later* treatment reduces genomic DNA contamination.
**FIG. 6A, FIG. 6B****,** **FIG. 6C, FIG. 6D****,** **FIG. 6E, and FIG. 6F** LK4-fractionation of blood reduces globin RNA contamination.

### DETAILED DESCRIPTION OF THE INVENTION

The inventors have devised new methods, in accordance with the first aspect of the invention as described above, for fractionating leukocytes and extracting mRNA from them. These methods involve the use of a leukocyte depletion filter, such as that produced by Pall Corporation, to capture leukocytes, followed by isolation of RNA from the captured leukocytes. The inventors have, in one embodiment, shown that a Pall leukocyte depletion filter, for example their filter designated "LK4," enables one to recover WBCs from whole blood for subsequent RNA extraction. Volumes of whole blood as large as 20 ml can be rapidly filtered (in approximately one minute) at the point of collection using a manual syringe format, which makes the method amenable to RNA extraction in the field. No centrifugation or mixing with other solutions is required. Further, the inventors have developed a closed-tube format for WBC fractionation, which offers minimal risk of exposure to blood-borne pathogens. WBC filtration is expected to perturb the mRNA profile of isolated leukocytes less than any of the alternative leukocyte fractionation methods, since it can be completed within 1 to 2 minutes of blood collection and does not subject the cells to the effects of centrifugal force. The yield and quality of RNA recovered using this method are excellent.

RNA*later* is a reagent developed at Ambion, which stabilizes RNA in tissue samples. RNA*later,* and various embodiments of it, are described in U.S. Patents No. 6,204,375 and 6,528,641, both entitled "Methods and Reagents for Preserving RNA in Cell and Tissue Samples". One embodiment of the invention involves a procedure for rapid fractionation of WBCs from whole blood and for rapid stabilization of mRNA in the fractionated cells using RNA*later.*

### Methods of Isolation of WBCs from Blood

There are a variety of manners in which to isolate WBCs from whole blood, many of which have been discussed above. However, in the context of the present invention, the method of isolation involves the use of a leukocyte depletion filter, which enables RBCs, blood plasma, and other non-WBC blood components to pass through while trapping WBCs.

Presently preferred filters for WBC isolation are the leukocyte depletion matrices sold by Pall Corporation, under the name Leukosorb^{®} Medium. Leukosorb is a fibrous medium that was originally designed for the depletion of WBCs from blood for transfusion. Descriptions of the Leukosorb products and their use may be found in U.S. Patent Nos.: 5,501,795, 5,100,564, 4,880,548, 4,923,620, 4,925,572, 5,229,012, and 5,344,561, as well as U.S. Patent Application No. 20030134417. In the studies discussed below, the LK4 Leukosorb filters were used.

### Methods of Isolating RNA from WBCs

There are many method for isolating RNA from cells, known to those of skill in the art and most or all of them are adaptable to the present invention.

Most methods for extracting total RNA from whole blood and fractionated leukocytes involve an initial lysis of the sample in guanidinum thiocyanate, a potent chaotrope that disrupts cell membranes and denatures proteins, including nucleases. One commonly used commercial reagent, TRIzol® (Life Technologies, Inc.), consists of a mixture of guanidinium salts and phenol. In a standard protocol, whole blood is mixed with three volumes of TRIzol reagent, then chloroform is added and the prep is centrifuged to separate it into organic and aqueous phases. The RNA is precipitated out of the aqueous phase with isopropanol and the pellet washed with ethanol to reduce contaminants.

Another common method for purifying RNA from blood involves solid-phase extraction onto a silica matrix. The blood sample is lysed in a guanidinium solution or other lysis solution, and the cell lysate is mixed with ethanol and applied to the silica filter, which binds RNA. The filter is washed to remove contaminants and the RNA is then eluted with water. A DNase digestion step is typically recommended in all RNA isolation methods to eliminate contaminating genomic DNA.

Other methods are based on lysis of whole blood in cationic detergents (Macfarlane and Dahle, 1997) or in lithium chloride/urea solutions.

Commercial products for isolating RNA from blood include the Ambion RiboPure-Blood kit (Ambion) and the PAXgene® system (PreAnalytix). The use of the RiboPure-Blood kit is described in the Examples below. In the PAXgene system, blood samples of ∼ 2.5 ml are drawn directly into evacuated blood collection tubes containing ∼7.5 ml of lysis solution. After storage of the sample at room temperature for at least 2 hours, the RNA is pelleted, washed, treated with protease, then the prep is mixed with ethanol and then purified over a silica filter as described above.

### Methods of Using RNA and DNA Isolated from WBCs

RNA and DNA isolated from WBCs in the manner of the invention may be used in any molecular biological technique involving RNA or DNA, as will be understood by those of skill in the art.

Some preferred embodiments of the invention involve expression profiling for research and/or diagnostic purposes, as described below. Other embodiments involve the use of RNA isolated from WBCs in reverse transcriptase-polymerase chain reaction protocols, such as those described in U.S. Patent Publication No. 20030170617, entitled "Crude biological derivatives competent for nucleic acid detection," by Pasloske. In one preferred embodiment of the invention, WBCs isolated using the leukocyte depletion filters described herein are treated with the methods described in U.S. Patent Publication No. 20030170617.

Other uses include analysis of levels of expression of mRNAs transcribed from one or several genes, analysis of global mRNA expression levels, analysis of expression levels of small endogenous and exogenous RNA molecules such as micro RNAs and small interfering RNAs, and discovery of and analysis of genetic polymorphisms including mutations and SNPs.

For example, assays such as Northern blots, RNase protection assays, S1 nuclease protection assays, and reverse transcription polymerase chain reaction (RT-PCR) may be used in analysis of levels of one or a few mRNAs. A particularly useful assay for one or several mRNA species is a modification of RT-PCR known as real-time RT-PCR (also known as quantitative RT-PCR). In general, these assays are either based on hybridization of an RNA sample to a labeled probe, for example to an RNA or DNA probe labeled by incorporation of a nucleotide coupled to a detectable moiety such as 32-P or biotin or digoxigenen; or the assays are based on conversion of target RNAs in the RNA sample to complementary DNA (cDNA) using retroviral reverse transcriptase enzyme(s), for example reverse transcriptase isolated from or derived from Maloney Murine Leukemia Virus (M-MuLV) or from Avian Myeloblastosis Virus (AMV) or from human immunodeficiency virus (HIV), with subsequent amplification of the cDNA target(s) using PCR or using RNA polymerase enzymes such as T7, T3, and Sp6 RNA polymerases.

### Methods of Expression Profiling

RNA recovered from fractionated blood cells using the present invention can be used as input for expression profiling experiments that aim to discover patterns of mRNA levels that correlate with disease, prognosis, and response to treatment.

In general, RNA used for expression profiling is converted to complementary DNA (cDNA) by enzymes having reverse transcriptase activity, and detectable moieties, for example fluorescently labeled dNTPs or biotinylated dNTPs, are incorporated into the cDNA during the reverse transcription step. The labeled cDNA is then hybridized to elements arrayed on a solid support, such that a detectable signal, for example fluorescence, is associated with elements that are specific for distinct mRNAs. The elements on the array can consist of oligonucleotides or cDNAs complementary to target mRNAs.

Requirements for input RNA are that it not contain inhibitors of reverse transcriptase or levels of genomic DNA which could interfere with specific detection of the desired mRNA targets.

Another undesirable contaminant in input RNA used for expression profiling from leukocytes is mRNA from the α- and β-globin genes. A complication of expression profiling using input RNA extracted from unfractionated whole blood is the presence of high numbers (relative to number of leukocytes) of immature red blood cells, known as reticulocytes, which contain high levels of mRNA for α- and β-globin. When RNA is extracted from whole blood and amplified for use in microarray expression profiling assays, the product of globin mRNA predominates in the amplified material and results in dramatically reduced sensitivity for detection of other mRNAs of interest. (Affymetrix Technical Notes, "Blood RNA Isolation Methods for GeneChip Expression Analysis"; "An Analysis of Blood Processing Methods to Prepare Samples for GeneChip Expression Profiling"). This globin mRNA contamination compromises use of the RNA as input for microarray profiling. A particular advantage of the current invention is that most reticulocytes are removed during the process of fractionating the blood over the leukocyte depletion filter.

### EXAMPLES

The following examples are included to further illustrate various embodiments of the invention. It should be appreciated by those of skill in the art that the techniques disclosed in the examples that follow represent techniques and/or compositions discovered by the inventor to function well in the practice of the invention, and thus can be considered to constitute preferred modes for its practice.

### Example 1

### Efficiency of Capture of Leukocytes on Leukocyte Depletion Matrix

A study was carried out to demonstrate the efficiency with which leukocytes are captured from whole blood using a prototype device. Six 10 ml samples of EDTA blood were passed through Pall Corporation LK4 filters and the filtrates were collected in fresh tubes. The filtrates were then centrifuged for 15 minutes at 3,200 rpm in a swinging bucket centrifuge in order to recover any WBCs that were not captured as a buffy coat fraction. Buffy coats were collected using disposable wide-bore transfer pipettes.

RNA was extracted from LK4 filters and from the corresponding buffy coat fractions using the Ambion RiboPure-Blood kit. Briefly, this protocol involves lysing the WBCs (which may be captured onto the leukocyte depletion matrix or which may be present in whole blood or in the buffy coat fraction of blood) in a guanidinium thiocyanate-based lysis solution, adding sodium acetate to reduce the pH of the preparation, adding low-pH phenol/chloroform and vortexing the preparation thoroughly and then storing it for 5 minutes at room temperature. The mixture is then centrifuged to separate the aqueous and organic phases, and the upper aqueous phase is removed to a new vessel and mixed with an appropriate volume of absolute ethanol, for example with one-half volume of absolute ethanol. The lower, organic phase, which contains most of the contaminating heme and plasma and cellular proteins, is discarded. The aqueous phase + ethanol mixture is then filtered through a silica membrane, where RNA binds to the silica matrix. The silica matrix is washed with a first wash solution, containing guanidinium and other proprietary components, followed by washing with a second wash solution containing ethanol and other proprietary components. The filtration and wash steps are accomplished by passing the solutions through the silica filter by centrifugation or by vacuum filtration. After the wash steps are completed, the silica filter is centrifuged to remove residual fluid and the filter is transferred to a second vessel, for example a 2 ml microfuge tube. The RNA is then eluted from the silica filter by applying preheated nuclease-free water containing 0.1 mM EDTA to the silica matrix and centrifuging the assembly to recover the RNA.

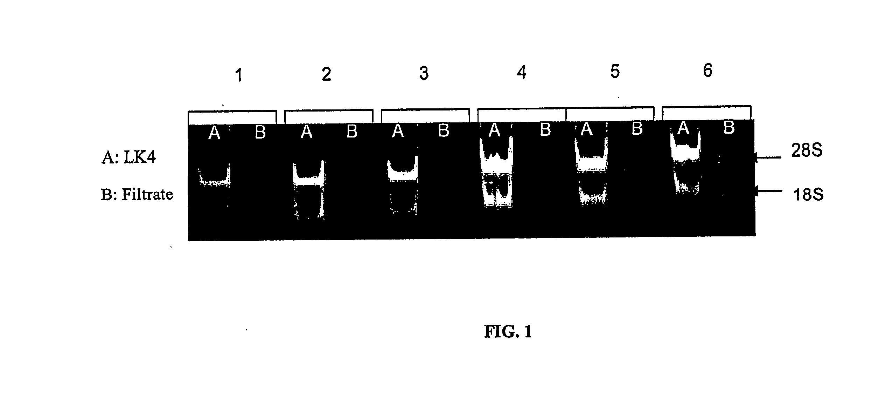

The RNA from each sample was eluted in 200 ul, and 15 ul of each prep was analyzed on a denaturing agarose gel in the presence of ethidium bromide, as shown in FIG. 1. Samples were loaded in pairs, with the LK4-recovered RNA in the first lane and the corresponding filtrate-recovered RNA loaded in the adjacent lane. The efficiency of WBC capture from whole blood using the filter matrix in this format ranged from ∼ 75 - 85%, as shown in FIG. 1. This efficiency is more than adequate to obtain sufficient RNA for use as input in microarray expression profiling experiments.

### Example 2

### High-quality RNA Can be Recovered from Filter Matrix-Fractionated Leukocytes

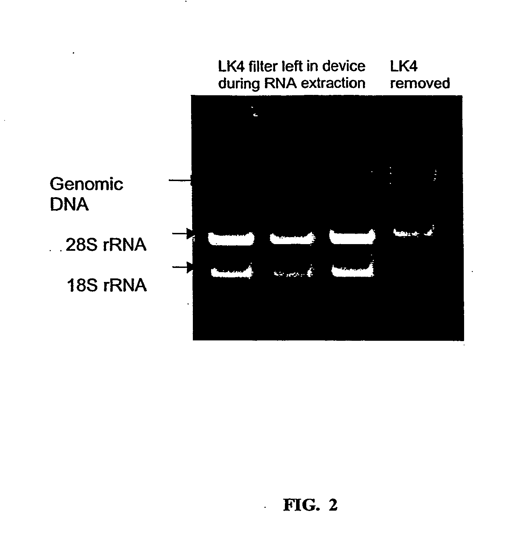

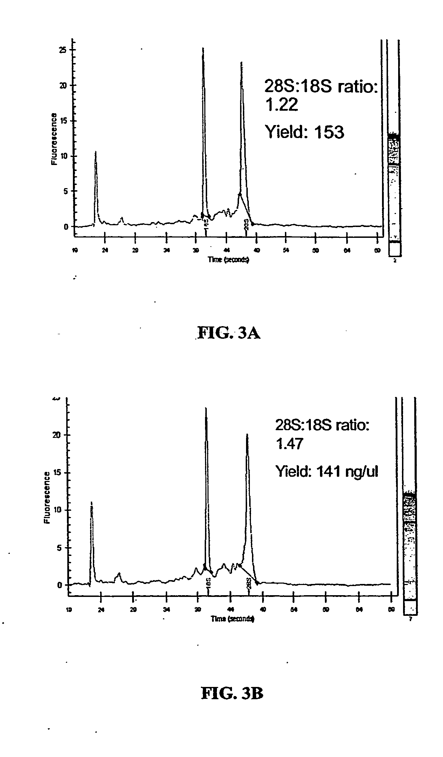

FIG. 2, FIG. 3A and FIG. 3B show the yield and quality of RNA recovered from LK4-fractionated cells using the methods of the present invention.

FIG. 2 shows analysis of the RNA via agarose gel electrophoresis. Anticoagulated blood (10 ml) was filtered through 2.5 cm-diameter circles of LK4 using one of two formats. In this study, the samples in the first 3 lanes were processed using a format in which the LK4 filter remains in the disposable syringe device and the extraction reagents are flushed through the filter to release the RNA. The sample in the last lane in FIG. 2 was recovered from blood processed using LK4 in a reusable syringe device format in which the filter was removed from the device for processing. The RNA from each sample was eluted in 200 µl of nuclease-free water containing 0.1 mM EDTA, and 15 µl of each prep was analyzed on a denaturing 1% agarose gel in the presence of ethidium bromide. Intact high-quality RNA is apparent as evidenced by the sharp bands of ethidium-staining material corresponding to the signature 18S (lower band) and 28S (upper band) ribosomal RNA. The RNA recovered using the format in which the LK4 filter is removed (lane 4 in FIG. 2) consistently showed a higher level of contamination with genomic DNA, compared to the alternative format, in which the filter is processed *in situ* (lanes 1-3 in FIG. 2). The purity of RNA recovered from the LK4-captured cells was also assessed by determining the UV- absorbance values at 260 nm and 280 nm; the 260:280 ratios spanned a narrow range from 1.9 - 2.1, indicative of highly pure RNA.

FIG. 3A and FIG. 3B show RNA analyzed *via* microfluidic separation on an Agilent 2100 Bioanalyzer. Analysis of RNA on the Agilent Bioanalyzer, for example, is particularly useful in regard to some embodiments of the invention, as this instrument provides a numerical value for the ratio of peak heights of the 18S and 28S rRNAs. The ∼5 kilobase 28S rRNA is more susceptible to degradation than the ∼ 2 kb 18S rRNA, and so higher 28S:18S rRNA values are associated with RNA which is more highly intact. Although a consensus 28S:18S rRNA value indicative of RNA of sufficient quality to use as input for microarray experiments is still emerging, it has been suggested that RNA should have a 28S:18S rRNA ratio of at least 1 to be used most successfully for this application. FIG. 3A and FIG. 3B show representative Agilent Bioanalyzer data for two RNA preps extracted from LK4-fractionated leukocytes using the format in which the filter is removed from the device for processing. These preps were treated with DNase 1 after elution. One microliter of each prep was mixed with a sample-loading solution containing a proprietary dye that binds specifically (or at least, preferentially) to RNA and was run on the Agilent chip. The 28S:18S rRNA peak height ratios are all well above the cut-off value of 1. In general, the yield of RNA from this type of prep, as determined by the Agilent software, ranges from ∼ 30 to 60 ug per 10 ml of blood.

### Example 3

### RNAlater Treatment Preserves RNA in Fractionated WBCs

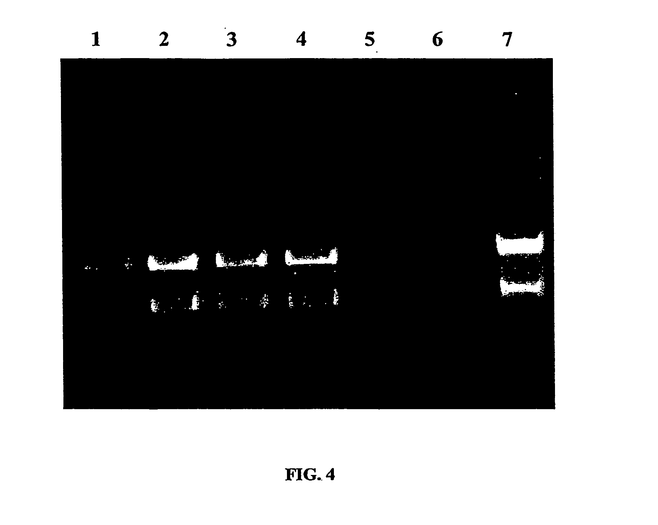

The data shown in Figure 4 demonstrate the benefit of treating LK4-captured cells with RNA*later* for preserving RNA intactness, as shown by more intensely-staining 18S and 28S ribosomal RNA bands in samples treated with RNA*later,* compared to untreated samples. A particular advantage of treating LK4-captured cells with RNA*later* is that said treatment allows the device containing the captured cells to be transported at ambient temperature from the point of sample collection to a distant-site lab for RNA extraction and analysis. Transporting samples at ambient temperature reduces the costs associated with RNA analysis.

To obtain these data, duplicate 10 ml EDTA blood samples from 3 healthy donors were filtered over LK4 leukocyte depletion matrix contained in disposable syringe filter units. One of each pair of samples was filtered over a disposable filter unit device having a design called "ring-molded", and the other was filtered through a disposable filter unit having an alternative design called "psf". After filtering the blood, the captured cells on some filters were treated with RNA*later* by passing 2 ml of RNA*later* through the LK4 matrix; the RNA*later* was delivered via a 3 ml syringe attached to the filter unit containing the LK4 matrix with captured cells.

The filter units containing the LK4 captured cells were removed from the syringes and shipped via overnight courier at ambient temperature from the sample collection point to the laboratory. RNA was extracted from the LK4-captured cells, eluted in 230 uL of water containing 0.1 mM EDTA, and 15 ul (∼ 6.5% of each prep) was mixed with 8 uL of denaturing gel-loading solution containing 10 ug/ml ethidium bromide and resolved by electrophoresis on a denaturing agarose gel. The samples in each lane were as follows: Lane 1-Donor #1, sample filtered through ring-molded device, treated with RNA*later;* Lane 2-Donor #1, sample filtered through psf device, treated with RNA*later;* Lane 3-Donor #2, sample filtered through ring-molded device, treated with RNA*later*; Lane 4-Donor #2, sample filtered through psf device, treated with RNA*later*; Lane 5-Donor #3, sample filtered through ring-molded device, NOT treated with RNA*later*; Lane 6-Donor #3, sample filtered through psf device, NOT treated with RNA*later*; and Lane 7-reference RNA sample, not processed at same time as other samples.

As can be seen in the data in FIG. 4, those samples not treated with RNA*later* had significantly degraded RNA, as opposed to those treated with RNA*later.* These data show that RNA*later* treatment results in significant advantages in the context of the invention.

### Example 4

### RNAlater Treatment Reduces Genomic DNA Contamination

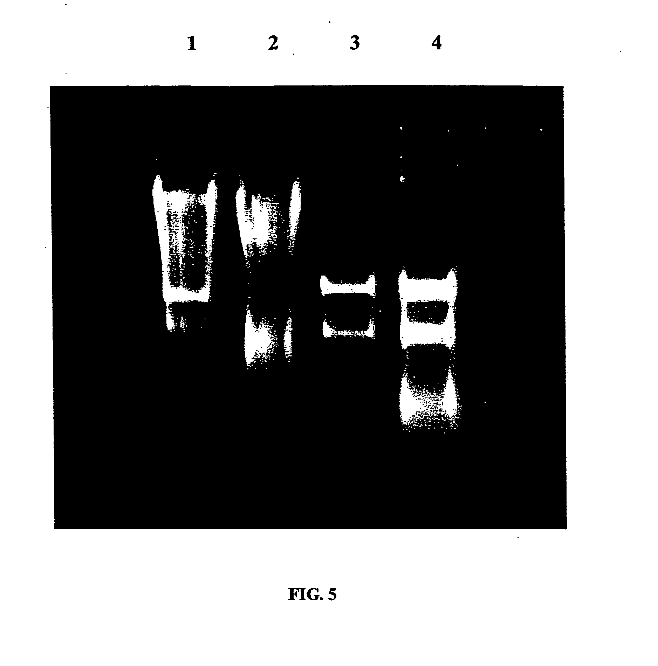

FIG. 5 shows the advantage of exposing the filtered leukocytes to RNA*later* to reduce genomic DNA contamination. All samples were from 10 ml of EDTA-blood filtered through a 2.5 cm-diameter LK4 filter and stored for 4 days in a room temperature as indicated. Approximately 7% of the RNA recovered from each prep was analyzed on a denaturing agarose gel. In lane 1, the filter was not treated. In lane 2, the filter was stored in 2.4 ml of guanidinium thiocyanate lysis solution. In lane 3, the filter was treated with 3 ml of RNA*later* before storage. Lane 4 shows a positive control RNA with the filter not treated, not stored, and treated with DNase post-elution. Note that there was severe degradation of RNA in the filter stored for 4 days at room temperature in GuSCN solution (Lane 2). Further, note that DNA contamination was greatly reduced in the sample (in Lane 3) treated with RNA*later* prior to extraction.

### Example 5

### Filter Matrix Fractionization of WBCs Reduces Globin mRNA Contaimination

A study was carried out to assess the extent to which LK4 fractionation reduces the undesired product of contaminating globin mRNAs in the amplified RNA (aRNA), compared to the amount of globin mRNA-derived amplified RNA from whole blood or from WBCs fractionated by centrifugation.

Several tubes of EDTA anticoagulated blood from a single donor were processed. Total RNA was extracted from LK4 fractionated WBCs, from whole blood, and from WBCs fractionated by centrifugation ("buffy coat" fractionated WBCs). The RNA from these three types of samples was used as input for linear amplification mediated by T7 RNA polymerase, using the Ambion MessageAmp™ kit. Non-blood-derived RNA from the kit (from the human HeLa cell line) was included as positive control. After first-strand and second-strand synthesis, the double-stranded cDNA was transcribed with T7 RNA polymerase and the aRNA (amplified RNA) reaction products were mixed with a proprietary RNA-binding fluorescent dye matrix and analyzed on the Agilent 2100 Bioanalyzer. This instrument separates heterogeneous RNA molecules according to size, with smaller molecules migrating faster than larger molecules.

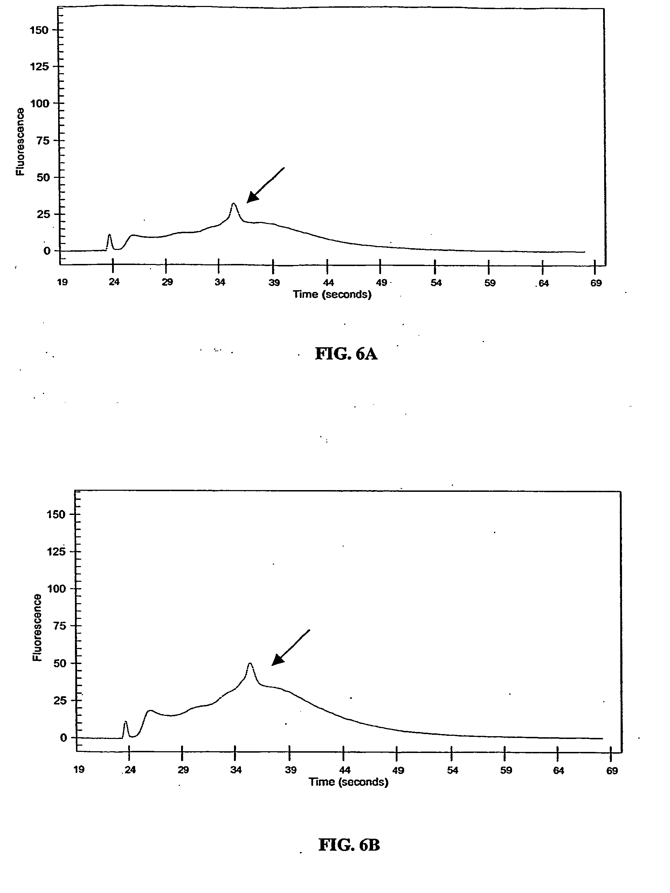

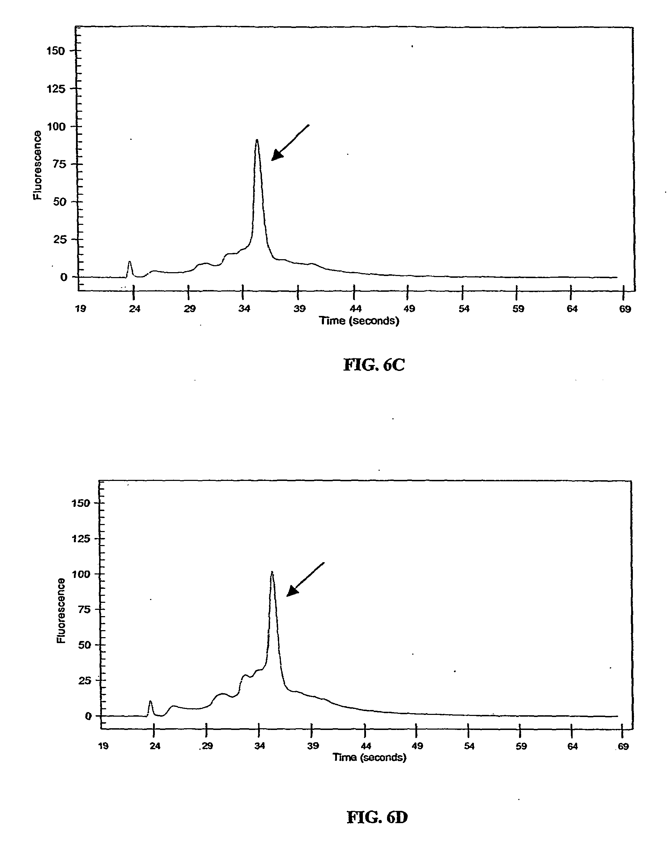

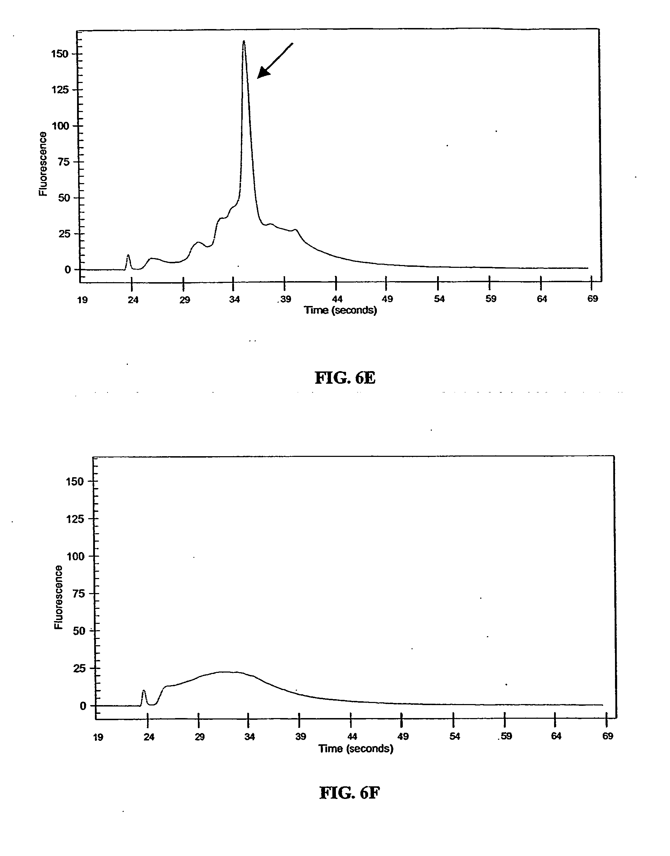

FIG. 6A, FIG. 6B, FIG. 6C, FIG. 6D, FIG. 6E, and FIG. 6F show data from this study. In each of these, the horizontal axis indicates time and the vertical axis indicates fluorescent intensity. The arrows in each panel indicate the "spike" of amplified RNA derived from α and β globin mRNAs (as described in the Affymetrix Technical Bulletin, "Globin Reduction Protocol: A Method for Processing Whole Blood RNA Samples for Improved Array Results"). The samples from the positive control HeLa RNA are not expected to have globin aRNA spikes, since these were not amplified from blood-derived RNA. The magnitude of the globin aRNA spike in FIG. 6A and FIG. 6B, which contain RNA amplified from LK4-fractionated WBCs, is significantly reduced compared to the magnitude of the globin aRNA spike in FIG. 6C and FIG. 6D, which contain aRNA from unfractionated whole blood. The globin aRNA spike is also dramatically lower in the LK4-fractionated samples compared to the globin aRNA spike in the buffy-coat fractionated sample in FIG. 6E. FIG. 6F contains aRNA from the HeLa cell line control RNA, and hence does not show any spike of globin product.

### Example 6

### Lysis of Reticulocytes to Reduce Globin mRNA Contamination

Further studies were carried out to demonstrate that if lysis of reticulocytes could reduce levels of α- and β-globin transcript in RNA preparations from blood.

RNA was extracted from whole blood (WB) or cells captured in a leukocyte depletion filter (LDF) that had previously been washed with a RBC lysis solution (150 mM NH₄Cl, 10mM NaHCO₃, pH: 7.0). Levels of contaminating RNA were accessed by one-step quantitative RT-PCR (qRT-PCR) using an input of 10ng of total RNA per reaction (as determined by UV absorbance readings a 260nm on a Nanodrop spectrophotometer). Amplification primers were specific to either α or β globin sequences, and reaction product was detected using Assays On Demand (FAM/Tet Taqman® probes and primers) from ABI according to the manufacturers protocol.

The results of these studies are shown in Table 1. Data shows reduced levels of mRNA (i.e. higher cycle threshold (Ct)) for α and β globin in LDF samples as compared to WB samples. The Ct decrease was calculated to be 4.28 for α-globin RNA and 5.54 for β-globin RNA. These values correspond to decreases globin RNAs of ∼20-fold and ∼48-fold respectively. Thus binding leukocytes to the depletion matrix and washing the matrix with a RBC lysis solution is highly effective in reduction globin RNA contamination.

**Table 1**

| Sample | Primer | Cycle threshold |
|---|---|---|
| LDF | α-globin | 11.69, 11.72 |
| WB | α-globin | 7.43, 7.42 |
| | | |
| LDF | β-globin | 13.23, 13.47 |
| WB | β-globin | 7.74, 7.89 |

### Example 7

### Exemplary Procedure for Isolating mRNA from Leukocytes

In a preferred embodiment of the method of the invention, a leukocyte depletion filter, for example an LK4 filter, is cut or provided which fits a syringe filter unit that allows the filter to be recovered after filtering the blood. This method has can be used to filter 40 ml or more of blood without filter clogging.

Exemplary steps are as follows:
1. Obtain a sample, for example 10 to 20 ml, of anticoagulated whole blood using any manner known, for example, using EDTA as an anticoagulant and collecting the blood in a standard evacuated tube, i.e. a Vacutainer tube.
2. Transfer the blood to a syringe, for example as 20 - 30 ml syringe or other appropriate receptacle. In some embodiments, this is done without opening the tube of blood, by using a vented spike inserted through the stopper of the blood collection tube and attached via a luer fitting to the syringe.
3. Attach a filter device containing a leukocyte depletion filter, for example a ∼ 2.5 cm diameter circle of filter, to the syringe and pass the blood through the filter. The inventors typically pass the blood through the filter at a rate such that it drips at ∼ 2 - 4 drops per second. The leukocyte-depleted blood can be collected in a waste container for disposal, or it can be collected and retained for use in other clinical assays. After all blood has passed through, pump ∼ 10 ml of air through the filter to flush out most of the residual blood (this will often come out as foamy material). This step fractionates the leukocytes onto the filter and concentrates the sample ∼ 50-fold. An alternative configuration for carrying out this step is to place the leukocyte depletion filter device between the vented spike inserted into the blood collection tube and the syringe used to draw the blood through the filter. In this case, the leukocytes will be collected on the distal face of the filter (with respect to the syringe), and the leukocyte-depleted blood will be transferred to the syringe and can then be disposed of.
4. (Optional) Treat the leukocyte depletion filter containing the captured leukocytes with a RBC lysis buffer. The RBC lysis buffer can be passed through the filter, and the elutant containing reticulocyte and erythrocyte lysate can then be discarded.
5. Treat the leukocyte depletion filter containing the captured leukocytes with RNA*later.* One way to do this is to attach a syringe containing RNA*later* to the filter device containing the filter and flush the RNA*later* through the filter. Typically 2 - 3 ml of RNA*later* is sufficient. All fluid can be flushed, but the passing of too much air through the filter should be avoided, to prevent drying the filter. The filter should remain damp with RNA*later.* An alternative way to do this is to insert the vented spike described above into a container with RNA*later* and aspirate the RNA*later* up through the filter into the syringe.
6. Open the device that houses the filter and remove the filter with forceps; place it into, for example, a 15 ml polypropylene disposable conical centrifuge tube (15 ml Falcon tube). The filter with RNA*later*-stabilized leukocytes may be stored at ambient temperature for at least one week before RNA isolation.
7. To extract the RNA, use the reagents from the Ambion RiboPure-Blood™ kit, or any other appropriate RNA extraction procedure. If using the Ambion RiboPure-Blood kit, the steps of isolation are roughly as follows. Obviously, the steps, amounts, and concentrations in these procedures are subject to modification or substitution by those of skill in the art.
   a. Add 2.4 ml of guanidinium thiocyanate lysis solution and 0.15 ml of sodium acetate solution to the 15 ml tube with the filter and shake for 30 seconds.
   b. Add 1.5 ml of acid-phenol/chloroform to the tube and shake for 30 seconds, then store the prep for 5 minutes at room temperature.
   c. Centrifuge the prep for 10 minutes at 3,200 rpm in a table-top centrifuge.
   d. Aspirate or pour off the aqueous phase into a second 15 ml tube and add one-half volume of ethanol and vortex to mix; volume of prep at this stage is ∼ 4.5 ml.
   e. Pass the prep over a silica filter in a microcentrifuge cartridge format (RNAqueous filter cartridge); this will require ∼ 6 passes of spinning for ∼ 10 seconds each, if using a centrifuge, but can be done more easily using a vacuum manifold or syringe format.
   f. Wash the filter with 0.7 ml of Wash Solution #1.
   g. Wash the filter twice with 0.7 ml each time of Wash Solution #2/3, then centrifuge the filter for 1 minute to dry it.
   h. Elute the RNA from the filter with ∼200 ∼l of Elution Solution preheated to ∼ 75 °C.
8. If desired, treat the RNA with DNase 1 to eliminate residual genomic DNA;
   inactivate the DNase with DNA-free resin (recommended method).

### Example 8

### Exemplary Closed System for Rapid Fractionation of WBCs

The present invention allows for a closed system for rapid fractionation of WBCs from whole blood.

For example, one can filter leukocytes from anticoagulated whole blood collected in standard evacuated tubes (Vacutainer® tubes, BectonDickinson). In this regard, the rubber closure of the tube is pierced by a vented transfer spike, which is attached via a luer fitting to the inlet port of a syringe filter device containing a 2.5 cm diameter circle of leukocyte depletion matrix.

A standard 10 ml disposable syringe is attached to the outlet port of the filter device, and the blood is drawn through the filter into the syringe. The filtrate does not show any hemolysis and can be retained for blood chemistry tests or other assays. Other configurations can also be used for filtering the blood, for example the blood can be poured into the syringe (after removing the plunger and attaching the leukocyte depletion matrix containing device) and filtered by positive pressure. In either case, RBC lysis solution may be passed through the filter, subsequent to collecting leukocytes from the blood, in order to remove remaining reticulocytes and erythrocytes. Transfer of a 10 ml blood sample through a 2.5 cm LK4 filter typically takes less than one minute.

Advantages of this method are that it is rapid, eliminates exposure to blood-borne pathogens, and does not require any equipment such as centrifuges. This latter consideration means that the method is amenable to use in field situations where electrical power is unavailable, and in situations where centrifugation is impractical, such as in B3 biohazard containment hoods with limited space for bulky equipment. It will be appreciated by those of skill in the art that alternative formats can also be used to pass whole blood through filters in closed systems or even in open systems, for example where the blood collection tube is opened and the blood is poured into a syringe barrel with a filter device attached.

In some embodiments of the invention, the filter media remains within a housing throughout the WBC fractionation and RNA extraction process. This format provides benefits of ease and contamination prevention in some applications. However, in other embodiments, the filter media may be removed from a housing after fractionation but before RNA extraction.

### References

Alcorta D, Preston G, Munger W, Sullivan P, Yang YY, Waga I, Jennette JC, and Falk R. Microarray studies of gene expression in circulating leukocytes in kidney disease. Exp Nephrol 10:139-149 (2002).
Alizadeh AA, (28 additional authors), Brown PO, and Staudt LM. Distinct types of diffuse large B-cell lymphome identifed by gene expression profiling. Nature 403:503-511 (2000).
Chomczynski P and Sacchi N. Single-step method of RNA isolation by acid guanidinium phenol chloroform extraction. Anal. Biochemistry 162:156-159 (1987).
Claes K, Poppe B, Machackova E, Coene I, Foretova L, De Paepe A, and Messiaen L. Differentiating pathogenic mutations from polymorphic alterations in the splice sites fo BRCA1 and BRCA2. Genes Chromosomes Cancer 37:314-320 (2003).
Cummings CA and Relman DA. Using DNA microarrays to study host-microbe interactions. Emerg Infect Disease 6:513-525 (2000).
Duvigneau JC, Hartl RT, Teinfalt M, and Gemeiner M. Delay in processing porcine whole blood affects cytokine expression. J Immunological Methods 272:11-21 (2003).
Ebner S, Neyer S, Hofer S, Nussbaumer W, Romani N, and Heufler C. Generation of large numbers of human dendritic cells from whole blood passaged through leukocyte removal filters: an alternative to standard buffy coats. J. Immunol Methods 252:93-104 (2001).
Grace MB, McLeland CB, and Blakely WF. Real-time quantitative RT-PCR assay of GADD45 gene expression changes as a biomarker for radiation biodosimetry. Int'l Journal of Radiation Biology, 78:1011-21 (2002).
Guhaniyogi J and Brewer G. Regulation of mRNA stability in mammalian cells. Gene 265:11-23 (2001).
Hartel C, Bein G, Muller-Steinhardt M, and Kluter H. Ex vivo induction of cytokine mRNA expression in human blood samples. J Immunological Methods 249:63-71 (2001).
Haskill S, Johnson C, Eierman D, Becker S, and Warren K. Adherence induces selective mRNA expression of monocyte mediators and proto-oncogenes. J Immunol 140:1690-1694 (1988).
Hodge DL, Schill WB, Wang JM, Blanca I, Reynolds DA, Ortaldo JR, and Young HA. IL-2 and IL-12 alter NK cell responsiveness to IFN-gamma-inducible protein 10 by down-regulating CXCR3 expression. J Immunol 168:6090-6098 (2002).
Kaufman SH. Antagonism between camptothecin and topoisomerase II-directed chemotherapeutic agents in a human leukemia cell line. Cancer Res 51:1129-1136 (1991).
Macfarlane DE and Dahle CE. Isolating RNA from clinical samples with Catrimox-14 and lithium chloride. J Clin Lab Anal 11:132-139 (1997).
Magalhaes MC, Serra TA, and Magalhaes MM. RNA synthesis inhibitors on young rat adrenal in primary culture. An ultrastructural study. Tissue Cell 19:167-175 (1987).
Moenner M, Hatzi E, and Badet J. Secretion of ribonucleases by normal and immortalized cells grown in serum-free culture conditions. In vitro Cell Cev Biol Anim 33:553 (1997).
Pahl A and Brune K. Stabilization of gene expression profiles in blood after phlebotomy. Clinical Chemistry 48:2251-2253 (2002).
Raghavan A, Ogilvie RL, Reilly C, Abelson ML, Raghavan S, Vasdewan J, Krathwohl M, and Bohjanen PR. Genome-wide analysis of mRNA decay in resting and activated primary human T lymphocytes. Nucleic Acid Res 30:5529-5538 (2002).
Rainen L, Oelmueller U, Jurgensen S, Wyrich R, Ballas C, Schram J, Herdman C, Bankaitis-Davis D, Nicholls N, Trollinger D, and Tryon V. Stabilization of mRNA expression in whole blood samples. Clinical Chemistry 48:1883-1890 (2002).
Ross ME, Zhou X, Song G, Shurtleff SA, Girtman K, Williams WK, Liu H-C, Mahfouz R, Raimondi SC, Lenny N, Patel A, and Downing, JR. Classification of pediatric acute lymphoblastic leukemia by gene expression profiling. Blood 102:2951-2959 (2002).
Scheuermann RH and Racila E. CD19 antigen in leukemia and lymphoma diagnosis and immunotherapy. Leuk Lymphoma 18:385 - 397. (1995).
Stahlberg A, Aman P, Ridell B, Mostad P, and Kubista M. Quantitative real-time PCR method for detection of B-lymphocyte monoclonality by comparison of kappa and lambda immunoglobulin light chain expression. Clinical Chemistry 49:51-59 (2003).
Staudt LM and Brown PO. Genomic views of the immune system. Annual Review Immunology 18:829-859 (2000).
Stordeur P, Zhou L, and Goldman M. Analysis of spontaneous mRNA cytokine production in peripheral blood. J Immunol Methods 261:195-197 (2002).
Tang Y, Aigang L., Aronow BJ, and Sharp FR. Blood Genomic Responses Differ After Stroke, Seizures, Hypoglycemia, and Hypoxia: Blood genomic fingerprints of disease. Ann Neurol 50:699-707 (2001).
Theophilus, BDM, RNA Isolation and characterization protocols, pp 39-41, edit. R. Rapley and DL Manning, Humana Press, Totowa NJ, 1998
Verdeil G, Puthier D, Nguyen C, Schmitt-Verhulst AM, and Auphan-Anezin N. Gene profiling approach to establish the molecular basis for partial versus full activation of naive CD8 T lymphocytes. Ann NY Acad Sci 975:68-76 (2002).
Weitkamp JH and Crowe JE. Blood Donor Leukocyte Reduction Filters as a Source of Human B Lymphocytes. BioTechniques 31:464-466 (2001).
Whitney AR, Diehn M, Popper SJ, Alizadeh AA, Boldrick JC, Relman DA, and Brown PO. Individuality and variation in gene expression patterns in human blood. PNAS 100:1896-1901 (2003).
Xiang CC, Chen M, Kozhich OA, Phan QN, Inman JM, Chen Y, and Brownstein MJ. Probe generation directly from small numbers of cells for DNA microarray studies. BioTechniques 34:386-393 (2003).
Yeoh, E-J, Ross, ME, Shurtleff, SA, Williams, WK, Patel, D., Mahfouz, R, (14 other coauthors), and Downing, JR. Classification, subtype discovery, and prediction of outcome in pediatric acute lymphoblastic leukemia by gene expression profiling. Cancer Cell March 2002, pp 133 - 143.
Zhu H, Cong JP, Mamtora G, Gingeras T, and Shenk T. Cellular gene expression altered by human cytomegalovirus: global monitoring with oligonucleotide arrays. PNAS 95:14470-14475 (1998).

## Claims

1. An *in vitro* method of obtaining a leukocyte lysate comprising RNA comprising fractionating leukocytes from whole blood by passing the blood through a leukocyte depletion filter and lysing the fractionated leukocytes to obtain a lysate comprising RNA.

2. The method of claim 1, wherein the leukocytes are comprised on the filter at the time they are lysed.

3. The method of claim 1, wherein the leukocytes are flushed from the filter prior to lysis.

4. The method of claim 2, wherein the leukocyte comprising filter is stored for a period of time prior to lysis of the leukocytes.

5. The method of claim 1, wherein the fractionated leukocytes are contacted with a lysis solution.

6. The method of claim 5, wherein the lysis solution comprises a detergent.

7. The method of claim 6, wherein the detergent is Triton X-100, Tween-20, SDS (sodium dodecyl sulfate), sarcosyl, or deoxycholic acid.

8. The method of claim 5, wherein the lysis solution contains a chaotropic agent.

9. The method of claim 8, wherein the chaotropic agent is a guanidinium salt.

10. The method of claim 9, wherein the guanidinium salt is guanidinum thiocyanate.

11. The method of claim 5, wherein the lysis solution comprises a ribonuclease inhibitor.

12. The method of claim 5, wherein the lysis solution comprises a protease.

13. The method of claim 12, wherein the protease is proteinase K.

14. The method of claim 1, further comprising extracting the RNA from the lysate.

15. The method of claim 14, wherein extracting the RNA is performed via an organic extraction.

16. The method of claim 15, wherein the organic extraction is a phenol/chloroform extraction.

17. The method of claim 14, further comprising extracting RNA and DNA from the lysate.

18. The method of claim 1, comprising, prior to lysis, treating the fractionated leukocytes with an RNA preservation composition comprising a salt that infiltrates the leukocytes and increases the stability of the RNA compared to the RNA in cells not treated with the preservation composition.

19. The method of claim 18, wherein the salt is a sulfate salt.

20. The method of claim 19, wherein the salt is ammonium sulfate.

21. The method of claim 18, wherein the final salt concentration in the preservation composition is between 10 g/100 ml and a saturating concentration.

22. The method of claim 20, wherein the salt is present in the preservation composition at a final concentration of between 20 g/100 ml and the saturating concentration of the salt.

23. The method of claim 20, wherein the salt is present in the preservation composition at a final concentration of between 30 g/100 ml and 80 g/100 ml.

24. The method of claim 18, wherein the RNA preservation composition comprises at least two salts.

25. The method of claim 24, wherein the total salt concentration is present in the preservation composition at a final concentration of between 20 g/100 ml and 100 g/100 ml.

26. The method of claim 18, wherein the fractionated leukocytes are comprised on the leukocyte depletion filter and the filter is contacted with the RNA preservation composition.

27. The method of claim 18, further comprising extracting RNA from the fractionated leukocytes with an organic extraction.

28. The method of claim 27, wherein the extracted RNA has less DNA contamination than would RNA extracted from fractionated leukocytes that were not treated with the RNA preservation medium.

29. The method of claim 1, further comprising treating the fractionated leukocytes with a solution to reduce reticulocyte contamination.

30. The method of claim 29, wherein the solution is a RBC elution buffer.

31. The method of claim 29, wherein the solution is a RBC lysis solution.

32. The method of claim 31, wherein the RBC lysis solution is a water or ammonium chloride lysis solution.

33. The method of claim 1, further defined as comprising:
fractionating leukocytes from blood by capturing them with a leukocyte depletion filter;
lysing the fractionated leukocytes to produce a lysate;
extracting the lysate with an organic solution to form organic and aqueous phases;
separating the organic and aqueous phases; and
isolating RNA from the aqueous phase.

34. The method of claim 1, further defined as comprising:
fractionating leukocytes from blood by capturing them with a leukocyte depletion filter;
treating the fractionated leukocytes with an RNA preservation composition comprising a salt that infiltrates the leukocytes, increasing the stability of the RNA;
lysing the fractionated leukocytes to produce a lysate;
extracting the lysate with an organic solution to form organic and aqueous phases;
separating the organic and aqueous phases; and
isolating RNA from the aqueous phase.

35. The method of claim 1, further defined as comprising:
fractionating leukocytes from blood by capturing them with a leukocyte depletion filter;
treating the fractionated leukocytes with an RNA preservation composition comprising a salt that infiltrates the leukocytes, increasing the stability of the RNA;
lysing the fractionated leukocytes to produce a lysate; and
isolating RNA from the lysate.

36. The method of claim 1, further defined as comprising:
fractionating leukocytes from blood by capturing them with a leukocyte depletion filter;
lysing the fractionated leukocytes to produce a lysate; and
isolating RNA from the lysate.

37. The method of claim 1, further defined as comprising:
fractionating leukocytes from blood by capturing them with a leukocyte depletion filter;
treating the fractionated leukocytes with a solution to reduce reticulocyte contamination;
lysing the fractionated leukocytes to produce a lysate;
extracting the lysate with an organic solution to form organic and aqueous phases;
separating the organic and aqueous phases; and
isolating RNA from the aqueous phase.

38. The method of claim 1, further defined as comprising:
fractionating leukocytes from blood by capturing them with a leukocyte depletion filter;
treating the fractionated leukocytes with a solution to reduce reticulocyte contamination;
treating the fractionated leukocytes with an RNA preservation composition comprising a salt that infiltrates the leukocytes, increasing the stability of the RNA;
lysing the fractionated leukocytes to produce a lysate;
extracting the lysate with an organic solution to form organic and aqueous phases;
separating the organic and aqueous phases; and
isolating RNA from the aqueous phase.

39. The method of claim 1, further comprising assaying for the presence or quantity of one or more RNAs in the lysate.

40. The method of claim 39, wherein assaying comprises a Northern blot, RNase protection assay, hybridization reaction, microarray analysis, or reverse transcriptase-polymerase chain reaction analysis.

41. The method of claim 40, wherein assaying comprises a reverse transcriptase-polymerase chain reaction further defined as real-time RT-PCR or endpoint RT-PCR.

42. The method of claim 40, wherein assaying comprises a microarray analysis.

43. The method of claim 42, wherein the microarray analysis comprises the use of a cDNA array, spotted oligonucleotide array, or *in-situ* synthesized oligonucleotide array.

44. A kit for extracting total RNA from leukocytes comprising:
a leukocyte depletion filter; and
a leukocyte lysis solution.

45. The kit of claim 44, wherein the leukocyte depletion filter is comprised in a carrier adapted to allow blood to be passed through the filter during use.

46. The kit of claim 45, wherein the carrier is adapted to be fitted to a syringe.

47. The kit of claim 44, further defined as adapted to function in a manner that allows whole blood to be moved from a closed container through the filter and then, as leukocyte-depleted blood, into a further closed container.

48. The kit of claim 44, further comprising a RBC lysis solution and/or a RBC elution buffer.

49. The kit of claim 44, further comprising an RNA preservation composition comprising a salt that infiltrates leukocytes and increases the stability of the RNA in the leukocytes.

50. The kit of claim 44, further comprising an organic extraction reagent.

51. The kit of claim 50, further comprising a solid-phase extraction matrix and reagents for washing the matrix to remove impurities before elution of the RNA.

## Patentansprüche

1. Ein In-vitro-Verfahren zur Gewinnung eines RNA umfassenden Leukozytenlysats, das das Fraktionieren von Leukozyten aus Vollblut mittels Durchfluss des Blutes durch einen Leukozyten-Depletionsfilter und Lysieren der fraktionierten Leukozyten zur Gewinnung eines RNA umfassenden Lysats umfasst.

2. Das Verfahren nach Anspruch 1, wobei die Leukozyten zum Zeitpunkt ihres Lysierens auf dem Filter enthalten sind.

3. Das Verfahren nach Anspruch 1, wobei die Leukozyten vor dem Lysieren von dem Filter gespült werden.

4. Das Verfahren nach Anspruch 2, wobei der die Leukozyten umfassende Filter vor dem Lysieren der Leukozyten für eine Zeitspanne gelagert wird.

5. Das Verfahren nach Anspruch 1, wobei die fraktionierten Leukozyten mit einer Lyselösung in Kontakt gebracht werden.

6. Das Verfahren nach Anspruch 5, wobei die Lyselösung ein Detergenz umfasst.

7. Das Verfahren nach Anspruch 6, wobei das Detergenz Triton X-100, Tween-20, SDS (Natriumdodecylsulfat), Sarcosyl oder Desoxycholsäure ist.

8. Das Verfahren nach Anspruch 5, wobei die Lyselösung ein chaotropes Mittel beinhaltet.

9. Das Verfahren nach Anspruch 8, wobei das chaotrope Mittel ein Guanidiniumsalz ist.

10. Das Verfahren nach Anspruch 9, wobei das Guanidiniumsalz Guanidiniumthiocyanat ist.

11. Das Verfahren nach Anspruch 5, wobei die Lyselösung einen Ribonukleaseinhibitor umfasst.

12. Das Verfahren nach Anspruch 5, wobei die Lyselösung eine Protease umfasst.

13. Das Verfahren nach Anspruch 12, wobei die Protease Proteinase K ist.

14. Das Verfahren nach Anspruch 1, das ferner das Extrahieren der RNA aus dem Lysat umfasst.

15. Das Verfahren nach Anspruch 14, wobei das Extrahieren der RNA über eine organische Extraktion durchgeführt wird.

16. Das Verfahren nach Anspruch 15, wobei die organische Extraktion eine Phenol/Chloroform-Extraktion ist.

17. Das Verfahren nach Anspruch 14, das ferner das Extrahieren der RNA und DNA aus dem Lysat umfasst.

18. Das Verfahren nach Anspruch 1, das vor dem Lysieren das Behandeln der fraktionierten Leukozyten mit einer RNA-Konservierungszusammensetzung umfasst, die ein Salz umfasst, das die Leukozyten infiltriert und die Stabilität der RNA im Vergleich zu der RNA in Zellen erhöht, die nicht mit der Konservierungszusammensetzung behandelt wurden.

19. Das Verfahren nach Anspruch 18, wobei das Salz ein Sulfatsalz ist.

20. Das Verfahren nach Anspruch 19, wobei das Salz Ammoniumsulfat ist.

21. Das Verfahren nach Anspruch 18, wobei die Endkonzentration des Salzes in der Konservierungszusammensetzung zwischen 10 g/ 100 ml und einer Sättigungskonzentration liegt.

22. Das Verfahren nach Anspruch 20, wobei das Salz in der Konservierungszusammensetzung bei einer Endkonzentration zwischen 20 g/100 ml und der Sättigungskonzentration des Salzes vorliegt.

23. Das Verfahren nach Anspruch 20, wobei das Salz in der Konservierungszusammensetzung bei einer Endkonzentration zwischen 30 g/100 ml und 80 g/ 100 ml vorliegt.

24. Das Verfahren nach Anspruch 18, wobei die RNA-Konservierungszusammensetzung mindestens zwei Salze umfasst.

25. Das Verfahren nach Anspruch 24, wobei die gesamte Salzkonzentration in der Konservierungszusammensetzung bei einer Endkonzentration zwischen 20 g/ 100 ml und 100 g/ 100 ml vorliegt.

26. Das Verfahren nach Anspruch 18, wobei die fraktionierten Leukozyten auf dem Leukozyten-Depletionsfilter enthalten sind und der Filter mit der RNA-Konservierungszusammensetzung in Kontakt gebracht wird.

27. Das Verfahren nach Anspruch 18, das ferner das Extrahieren der RNA aus den fraktionierten Leukozyten mit einer organischen Extraktion umfasst.

28. Das Verfahren nach Anspruch 27, wobei die extrahierte RNA eine geringere DNA-Verunreinigung aufweist als RNA aufweisen würde, die aus fraktionierten Leukozyten extrahiert wurde, die nicht mit dem RNA-Konservierungsmedium behandelt wurden.

29. Das Verfahren nach Anspruch 1, das ferner das Behandeln der fraktionierten Leukozyten mit einer Lösung umfasst, um die Retikulozyten-Verunreinigung zu verringern.

30. Das Verfahren nach Anspruch 29, wobei die Lösung ein RBC-Elutionspuffer ist.

31. Das Verfahren nach Anspruch 29, wobei die Lösung eine RBC-Lyselösung ist.

32. Das Verfahren nach Anspruch 31, wobei die RBC-Lyselösung eine Wasser- oder Ammoniumchlorid-Lyselösung ist.

33. Das Verfahren nach Anspruch 1, das ferner folgendes umfasst:
Fraktionieren von Leukozyten aus Blut, indem sie mit einem Leukozyten-Depletionsfilter eingefangen werden;
Lysieren der fraktionierten Leukozyten zur Erzeugung eines Lysats;
Extrahieren des Lysats mit einer organischen Lösung zur Bildung von organischen und wässrigen Phasen;
Trennen der organischen und wässrigen Phasen; und
Isolieren der RNA aus der wässrigen Phase.

34. Das Verfahren nach Anspruch 1, das ferner folgendes umfasst:
Fraktionieren von Leukozyten aus Blut, indem sie mit einem Leukozyten-Depletionsfilter eingefangen werden;
Behandeln der fraktionierten Leukozyten mit einer RNA-Konservierungszusammensetzung, die ein Salz umfasst, das die Leukozyten infiltriert, wobei die Stabilität der RNA erhöht wird;
Lysieren der fraktionierten Leukozyten zur Erzeugung eines Lysats;
Extrahieren des Lysats mit einer organischen Lösung zur Bildung von organischen und wässrigen Phasen;
Trennen der organischen und wässrigen Phasen; und
Isolieren der RNA aus der wässrigen Phase.

35. Das Verfahren nach Anspruch 1, das ferner folgendes umfasst:
Fraktionieren von Leukozyten aus Blut, indem sie mit einem Leukozyten-Depletionsfilter eingefangen werden;
Behandeln der fraktionierten Leukozyten mit einer RNA-Konservierungszusammensetzung, die ein Salz umfasst, das die Leukozyten infiltriert, wobei die Stabilität der RNA erhöht wird;
Lysieren der fraktionierten Leukozyten zur Erzeugung eines Lysats; und
Isolieren der RNA aus dem Lysat.

36. Das Verfahren nach Anspruch 1, das ferner folgendes umfasst:
Fraktionieren von Leukozyten aus Blut, indem sie mit einem Leukozyten-Depletionsfilter eingefangen werden;
Lysieren der fraktionierten Leukozyten zur Erzeugung eines Lysats; und
Isolieren der RNA aus dem Lysat.

37. Das Verfahren nach Anspruch 1, das ferner folgendes umfasst:
Fraktionieren von Leukozyten aus Blut, indem sie mit einem Leukozyten-Depletionsfilter eingefangen werden;
Behandeln der fraktionierten Leukozyten mit einer Lösung zur Verringerung der Retikulozyten-Verunreinigung;
Lysieren der fraktionierten Leukozyten zur Erzeugung eines Lysats;
Extrahieren des Lysats mit einer organischen Lösung zur Bildung von organischen und wässrigen Phasen;
Trennen der organischen und wässrigen Phasen; und
Isolieren der RNA aus der wässrigen Phase.

38. Das Verfahren nach Anspruch 1, das ferner folgendes umfasst:
Fraktionieren von Leukozyten aus Blut, indem sie mit einem Leukozyten-Depletionsfilter eingefangen werden;
Behandeln der fraktionierten Leukozyten mit einer Lösung zur Verringerung der Retikulozyten-Verunreinigung;
Behandeln der fraktionierten Leukozyten mit einer RNA-Konservierungszusammensetzung, die ein Salz umfasst, das die Leukozyten infiltriert, wobei die Stabilität der RNA erhöht wird;
Lysieren der fraktionierten Leukozyten zur Erzeugung eines Lysats;
Extrahieren des Lysats mit einer organischen Lösung zur Bildung von organischen und wässrigen Phasen;
Trennen der organischen und wässrigen Phasen; und
Isolieren der RNA aus der wässrigen Phase.

39. Das Verfahren nach Anspruch 1, das ferner das Testen auf das Vorhandensein oder die Menge von einer oder mehr als einer RNA in dem Lysat umfasst.

40. Das Verfahren nach Anspruch 39, wobei das Testen einen Northern-Blot, einen RNase-Schutztest, eine Hybridisierungsreaktion, eine Mikroarrayanalyse oder eine Analyse mittels Reverse-Transkriptase-Polymerasekettenreaktion umfasst.

41. Das Verfahren nach Anspruch 40, wobei das Testen eine Reverse-Transkriptase-Polymerasekettenreaktion umfasst, die ferner als Echtzeit-RT-PCR oder Endpunkt-RT-PCR definiert ist.

42. Das Verfahren nach Anspruch 40, wobei das Testen eine Mikroarrayanalyse umfasst.

43. Das Verfahren nach Anspruch 42, wobei die Mikroarrayanalyse die Verwendung eines cDNA-Arrays, eines gepunkteten Oligonukleotidarrays oder eines in-situ synthetisierten Oligonukleotidarrays umfasst.

44. Ein Kit zum Extrahieren von Gesamt-RNA aus Leukozyten, das folgendes umfasst:
einen Leukozyten-Depletionsfilter; und
eine Leukozyten-Lyselösung.

45. Das Kit nach Anspruch 44, wobei der Leukozyten-Depletionsfilter in einem Träger enthalten ist, der angepasst ist, um im Gebrauch den Durchfluss des Blutes durch den Filter zu gestatten.

46. Das Kit nach Anspruch 45, wobei der Träger zum Aufsetzen auf eine Spritze angepasst ist.

47. Das Kit nach Anspruch 44, das ferner angepasst ist, um auf eine derartige Art und Weise zu funktionieren, die das Bewegen von Vollblut aus einem geschlossenen Behälter durch den Filter und dann als Leukozytenverarmtes Blut in einen weiteren geschlossenen Behälter gestattet.

48. Das Kit nach Anspruch 44, das ferner eine RBC-Lyselösung und/oder einen RBC-Elutionspuffer umfasst.

49. Das Kit nach Anspruch 44, das ferner eine RNA-Konservierungszusammensetzung umfasst, die ein Salz umfasst, das die Leukozyten infiltriert und die Stabilität der RNA in den Leukozyten erhöht.

50. Das Kit nach Anspruch 44, das ferner ein organisches Extraktionsreagenz umfasst.

51. Das Kit nach Anspruch 50, das ferner eine Festphasen-Extraktionsmatrix und Reagenzien zum Waschen der Matrix umfasst, um vor der Elution der RNA Verunreinigungen zu entfernen.

## Revendications

1. Procédé *in vitro* d'obtention d'un lysat de leucocytes comprenant de l'ARN, comprenant le fractionnement de leucocytes provenant de sang total par passage du sang à travers un filtre pour la déplétion leucocytaire et la lyse des leucocytes fractionnés pour obtenir un lysat comprenant de l'ARN.

2. Procédé selon la revendication 1, dans lequel les leucocytes sont inclus sur le filtre au moment où ils sont lysés.

3. Procédé selon la revendication 1, dans lequel les leucocytes sont chassés du filtre avant la lyse.

4. Procédé selon la revendication 2, dans lequel le filtre comprenant les leucocytes est stocké pendant une période de temps avant la lyse des leucocytes.

5. Procédé selon la revendication 1, dans lequel les leucocytes fractionnés sont mis en contact avec une solution de lyse.

6. Procédé selon la revendication 5, dans lequel la solution de lyse comprend un détergent.

7. Procédé selon la revendication 6, dans lequel le détergent est le Triton X-100, le Tween-20, le SDS (dodécyl sulfate de sodium), le sarcosyle ou l'acide désoxycholique.

8. Procédé selon la revendication 5, dans lequel la solution de lyse contient un agent chaotropique.

9. Procédé selon la revendication 8, dans lequel l'agent chaotropique est un sel de guanidinium.

10. Procédé selon la revendication 9, dans lequel le sel de guanidinium est le thiocyanate de guanidinium.

11. Procédé selon la revendication 5, dans lequel la solution de lyse comprend un inhibiteur de ribonucléase.

12. Procédé selon la revendication 5, dans lequel la solution de lyse comprend une protéase.

13. Procédé selon la revendication 12, dans lequel la protéase est la protéinase K.

14. Procédé selon la revendication 1, comprenant en outre l'extraction de l'ARN à partir de lysat.

15. Procédé selon la revendication 14, dans lequel l'extraction de l'ARN est effectuée au moyen d'une extraction organique.

16. Procédé selon la revendication 15, dans lequel l'extraction organique est une extraction au phénol/chloroforme.

17. Procédé selon la revendication 14, comprenant en outre l'extraction de l'ARN et de l'ADN à partir du lysat.

18. Procédé selon la revendication 1, comprenant, avant la lyse, le traitement des leucocytes fractionnés par une composition de conservation de l'ARN comprenant un sel qui infiltre les leucocytes et augmente la stabilité de l'ARN par comparaison avec l'ARN dans des cellules non traitées par la composition de conservation.

19. Procédé selon la revendication 18, dans lequel le sel est un sel sulfate.

20. Procédé selon la revendication 19, dans lequel le sel est le sulfate d'ammonium.

21. Procédé selon la revendication 18, dans lequel la concentration finale de sel dans la composition de conservation est entre 10 g/100 ml et une concentration de saturation.

22. Procédé selon la revendication 20, dans lequel le sel est présent dans la composition de conservation à une concentration finale d'entre 20 g/100 ml et la concentration de saturation du sel.

23. Procédé selon la revendication 20, dans lequel le sel est présent dans la composition de conservation à une concentration finale d'entre 30 g/100 ml et 80 g/100 ml.

24. Procédé selon la revendication 18, dans lequel la composition de conservation de l'ARN comprend au moins deux sels.

25. Procédé selon la revendication 24, dans lequel la concentration totale de sel est présente dans la composition de conservation à une concentration finale d'entre 20 g/100 ml et 100 g/100 ml.

26. Procédé selon la revendication 18, dans lequel les leucocytes fractionnés sont inclus sur le filtre pour la déplétion leucocytaire et le filtre est mis en contact avec la composition de conservation de l'ARN.

27. Procédé selon la revendication 18, comprenant en outre l'extraction de l'ARN à partir des leucocytes fractionnés par une extraction organique.

28. Procédé selon la revendication 27, dans lequel l'ARN extrait a moins de contamination par l'ADN que ne l'aurait l'ARN extrait de leucocytes fractionnés qui n'auraient pas été traités par le milieu de conservation de l'ARN.

29. Procédé selon la revendication 1, comprenant en outre le traitement des leucocytes fractionnés par une solution pour réduire la contamination par les réticulocytes.

30. Procédé selon la revendication 29, dans lequel la solution est un tampon d'élution des globules rouges.

31. Procédé selon la revendication 9, dans lequel la solution est une solution de lyse des globules rouges.

32. Procédé selon la revendication 31, dans lequel la solution de lyse des globules rouges est une solution de lyse aqueuse ou de lyse de chlorure d'ammonium.

33. Procédé selon la revendication 1, défini en outre comme comprenant :
- le fractionnement des leucocytes à partir du sang par capture de ceux-ci par un filtre pour la déplétion ;
- la lyse des leucocytes fractionnés pour produire un lysat ;
- l'extraction du lysat par une solution organique pour former des phases organique et aqueuse ;
- la séparation des phases organique et aqueuse ; et
- l'isolement de l'ARN à partir de la phase aqueuse.

34. Procédé selon la revendication 1, défini en outre comme comprenant :
- le fractionnement des leucocytes à partir du sang par capture de ceux-ci par un filtre pour la déplétion leucocytaire ;
- le traitement des leucocytes fractionnés par une composition de conservation de l'ARN, comprenant un sel qui infiltre les leucocytes, augmentant la stabilité de l'ARN ;
- la lyse des leucocytes fractionnés pour produire un lysat ;
- l'extraction de lysat par une solution organique pour former des phases organique et aqueuse ;
- la séparation des phases organique et aqueuse ; et
- l'isolement de l'ARN à partir de la phase aqueuse.

35. Procédé selon la revendication 1, défini en outre comme comprenant :
- le fractionnement des leucocytes à partir du sang par capture de saisie par un filtre pour la déplétion leucocytaire ;
- le traitement des leucocytes fractionnés par une composition de conservation de l'ARN, comprenant un sel qui infiltre les leucocytes, augmentant la stabilité de l'ARN ;
- la lyse des leucocytes fractionnés pour produire un lysat ; et
- l'isolement de l'ARN à partir du lysat.

36. Procédé selon la revendication 1, défini en outre comme comprenant :
- le fractionnement des leucocytes à partir du sang par capture de ceux-ci par un filtre pour la déplétion leucocytaire ;
- la lyse des leucocytes fractionnés pour produire un lysat ; et
- l'isolement de l'ARN à partir du lysat.

37. Procédé selon la revendication 1, défini en outre comme comprenant :
- le fractionnement des leucocytes à partir du sang par capture de ceux-ci par un filtre pour la déplétion leucocytaire ;
- le traitement des leucocytes fractionnés par une solution pour réduire la contamination par les réticulocytes ;
- la lyse des leucocytes fractionnés pour produire un lysat ;
- l'extraction du lysat par une solution organique pour former des phases organique et aqueuse ;
- la séparation des phases organique et aqueuse ; et
- l'isolement de l'ARN à partir de la phase aqueuse.

38. Procédé selon la revendication 1, défini en outre comme comprenant :
- le fractionnement des leucocytes à partir du sang par capture de ceux-ci par un filtre pour la déplétion leucocytaire ;
- le traitement des leucocytes fractionnés par une solution pour réduire la contamination par les réticulocytes ;
- le traitement des leucocytes fractionnés par une composition de conservation de l'ARN, comprenant un sel qui infiltre les leucocytes, augmentant la stabilité de l'ARN ;
- la lyse des leucocytes fractionnés pour produire un lysat ;
- l'extraction du lysat par une solution organique pour former des phases organique et aqueuse ;
- la séparation des phases organique et aqueuse ; et
- l'isolement de l'ARN à partir de la phase aqueuse.

39. Procédé selon la revendication 1, comprenant en outre la conduite d'un essai pour la présence ou la quantité d'un ou plusieurs ARN dans le lysat.

40. Procédé selon la revendication 39, dans lequel l'essai comprend un transfert de Northern, un essai de protection à la RNase, une réaction d'hybridation, une analyse par microréseau ou une analyse par réaction en chaîne par polymérase-transcriptase inverse.

41. Procédé selon la revendication 40, dans lequel la conduite de l'essai comprend une réaction en chaîne par polymérase-transcriptase inverse encore définie comme RT-PCR en temps réel ou RT-PCR à point de fin de titrage.

42. Procédé selon la revendication 40, dans lequel la conduite de l'essai comprend une analyse par microréseau.

43. Procédé selon la revendication 42, dans lequel l'analyse par microréseau comprend l'utilisation d'un réseau d'ADNc, d'un réseau d'oligonucléotides par taches, ou d'un réseau d'oligonucléotides synthétisé in situ.

44. Coffret pour l'extraction d'ARN total à partir de leucocytes comprenant :
- un filtre pour la déplétion leucocytaire ; et
- une solution de lyse des leucocytes.

45. Coffret selon la revendication 44, dans lequel le filtre pour la déplétion leucocytaire se compose d'un support apte à permettre à du sang de passer à travers le filtre pendant l'utilisation.

46. Coffret selon la revendication 45, dans lequel le support est apte à être adapté à une seringue.

47. Coffret selon la revendication 44, défini en outre comme apte à fonctionner d'une manière qui permet à du sang total d'être déplacé d'un conteneur fermé à travers le filtre puis, comme sang appauvri en leucocytes, dans un autre conteneur fermé.

48. Coffret selon la revendication 44, comprenant en outre une solution de lyse des globules rouges et/ou un tampon d'élution des globules rouges.

49. Coffret selon la revendication 44, comprenant en outre une composition de conservation de l'ARN comprenant un sel qui infiltre les leucocytes et augmente la stabilité de l'ARN dans les leucocytes.

50. Coffret selon la revendication 44, comprenant en outre un réactif d'extraction organique.

51. Coffret selon la revendication 50, comprenant en outre une matrice d'extraction en phase solide des réactifs pour laver la matrice afin d'éliminer les impuretés avant élution de l'ARN.
